# EUROPÄISCHE PATENTSCHRIFT

(11) **EP 2 131 670 B1**
(45) Veröffentlichungstag und Bekanntmachung des Hinweises auf die Patenterteilung: **29.12.2010**
(21) Anmeldenummer: 08716073.5
(22) Anmeldetag: 27.02.2008
(51) Int. Cl.: A23K 1/16, A23K 1/18, A61P 1/14

(54) **SÜSSSTOFFHALTIGE FUTTERMITTEL UND FUTTERMITTELZUSÄTZE**
SWEETENER-CONTAINING ANIMAL FOOD AND ANIMAL FOOD ADDITIVES
ALIMENTS POUR ANIMAUX À BASE D'ÉDULCORANT ET ADDITIFS POUR ALIMENTS POUR ANIMAUX

(30) Priorität: 12.04.2007 AT 5672007
(43) Veröffentlichungstag der Anmeldung: 16.12.2009
(73) Patentinhaber: Roth, Hermann, 65343 Eltville (DE)
(72) Erfinder: Roth, Hermann, 65343 Eltville (DE)
(74) Vertreter: Puchberger, Peter
(86) Internationale Anmeldenummer: PCT/EP2008/001539
(87) Internationale Veröffentlichungsnummer: WO 2008/125166

(56) Entgegenhaltungen:
- FR-A- 2 568 104
- GB-A- 2 185 674
- GB-A- 2 200 527
- US-A- 3 657 424
- US-B1- 6 251 464
- MELLOR, S.: "CITRUS EXTRACT- NATURAL CHOICE" FEED MIX, Bd. 10, 2002, Seiten 28-31, XP002483488 NLDOETINCHEM
- KAWAGUCHI, KIICHIRO; KIKUCHI, SEI-ICHI; HASUNUMA, RYOICHI; MARUYAMA, HIROKO; RYLL, ROLAND; KUMAZAWA, YOSHIO:: "Suppression of infection-induced endotoxin shock in mice by a citrus flavanone naringin" PLANTA MEDICA, Bd. 70, Nr. 1, 2004, Seiten 17-22, XP002483489 DETHIEME, STUTTGART
- KRE CMAN V, SKOTTOV N, WALTEROV D, ULRICHOV J, SIMNEK V (1998) M, DELGADO-LICON E, AYALA ALM, KATRICH E, TRAKHTENBERG S: "Changes in plasma lipid and antioxidant activity in rats as a result of naringin and red grapefruit supplementation" JOURNAL OF AGRICULTURAL AND FOOD CHEMISTRY., Bd. 53, 2005, Seiten 3223-3228, XP002483532 USAMERICAN CHEMICAL SOCIETY. WASHINGTON.

## Beschreibung

Die vorliegende Erfindung betrifft süßstoffhaltige Futtermittel bzw. Futtermittelzusätze.

Mittel zum Süßen werden bereits seit Jahren mit Erfolg in der Tieremährung, insbesondere in der Jungtierfütterung, verwendet. Das bekannteste dieser Mittel ist Zucker. Dieser zeigt aber erst ab Konzentrationen von über 3-5 % einen wirklich süßenden Effekt. Aufgrund dieses Umstandes und wegen technologischer Restriktionen hat sich der Einsatz synthetischer Süßstoffe insbesondere in der Ferkelfütterung durchgesetzt. Die wesentliche Quelle für die Süße ist dabei zumeist Na-Saccharin. Dieses zeichnet sich durch hohe Süßkraft aus, d.h. die Süßkraft von 1 kg Saccharin entspricht jener von ca. 300-500 kg Zucker. Saccharin hat aber einen metallischen Nachgeschmack, weswegen es zumeist mit anderen Süßstoffen kombiniert wird, um diesen Nachgeschmack zu maskieren. Eine alleinige Fütterung von Saccharin kann bei hoher Dosierung aufgrund des Nachgeschmackes sogar eine Depression der Futteraufnahme bewirken.

Ein sehr weit verbreiteter Süßstoff ist Neohesperidin-Dihydrochalcon (NHDC). Neohesperidin kann aus Zitrusfrüchten, bevorzugt aus Bitterorangen, gewonnen und nach bekannten Verfahren in NHDC übergeführt werden. NHDC hat einen lakritz-/mentholartigen Nachgeschmack. Es weist eine 2-4 Mal höhere Süßkraft als Saccharin auf. Für den Einsatz im Tierfutter spricht aber neben der hohen Süßkraft die synergistische Wirkung im Süßungsprofil mit Saccharin. NHDC maskiert den metallischen Nachgeschmack von Saccharin und verlängert das süße Gefühl auf der Zunge, wird aber aufgrund seines Nachgeschmacks und seines hohen Preises nicht alleine eingesetzt.

Die US-PS 6 251 464 beschreibt eine pulverförmige Süßstoffzusammensetzung für Tierfutter für Jungtiere, wobei diese einen Süßstoff und einen Süßstoffverstärker aufweisende Zusammensetzung in Form einer innigen Mischung der Komponenten mit im Wesentlichen gleichförmiger Verteilung von Süßstoff und Süßstoffverstärker in einem für alle Teilchen der Zusammensetzung konstanten Verhältnis vorliegt. Der in der Zusammensetzung enthaltene Süßstoff ist aus Na- oder Ca-Saccharinat, Saccharin, Aspartylphenylalanin, Acesulfam, Cyclamaten, Steviosid und deren Mischungen ausgewählt, und der Süßstoffverstärker ist aus Neohesperidin-Dihydrochalcon, Thaumatin, Glycerrhizin und deren Mischungen ausgewählt.

Bei Fütterungsversuchen in der Ferkelaufzucht unter Verwendung herkömmlicher Futtermittel, die Neohesperidin-Dihydrochalcon (NHDC) in Kombination mit Saccharin enthalten, zeigt sich, dass NHDC wohl in der Lage ist, den metallischen Nachgeschmack von Saccharin zu maskieren, sodass besserer Futterverzehr und besserer Tageszuwachs resultieren, doch bleibt der Aufwand an Futter unbeeinflusst. Die GB-A-2185674 beschreibt ein Ferkelfuttermittel mit Gehalten an Saccharin und Thaumatin.

Die vorliegende Erfindung schlägt die Verwendung einer Süßstoffkombination, die zumindest Naringin-DC und ein Saccharin enthält, zur Appetit- und Leistungsförderung bei Nutztieren vor.

Naringin-DC ist ein relativ neuer Süßstoff. Es hat je Kilogramm eine Süßkraft, die der von etwa 500 - 800 kg Zucker entspricht. Es zeichnet sich durch einen lang anhaltenden süßen, neutralen Geschmack aus. Erfindungsgemäß zeigt sich, dass es in der Lage ist, den bitteren Beigeschmack von Saccharin zu überdecken, ohne nachteilige Futteraufnahmedepressionen zu verursachen. Naringin kann aus Grapefruit gewonnen und nach bekannten Verfahren in Naringin-DC übergeführt werden. Es ist bekannt, dass bei einer Reihe von Medikamenten (z.B. Cyclosporin, Felodipin) die Resorption verbessert wird, wenn diese zusammen mit Grapefruitsaft genommen werden. Hier wird im Bereich der Medizin diskutiert, ob die Dosierung von Medikamenten reduziert werden kann, wenn sie zusammen mit Grapefruitsaft verabreicht werden.

Das Saccharin in der erfindungsgemäß verwendeten Süßstoffkombination kann aus Saccharin und seinen Alkalimetall- oder Erdalkalimetallsalzen, wie beispielsweise Na-, K- oder Ca-Saccharin, ausgewählt sein. Im Rahmen der vorliegenden Erfindung werden unter Saccharin und/oder Saccharin-Salzen sowohl wasserfreie Formen als auch Hydrate verstanden.

Erfindungsgemäß kann die appetit- und leistungsfördernde Süßstoffkombination mindestens einen weiteren Süßstoff enthalten, der vorzugsweise aus Stevia-Extrakten und Thaumatin ausgewählt ist.

Der Ausdruck "Stevia-Extrakt", wie er in der vorliegenden Erfindung verwendet wird, umfasst die von Pflanzen der Gattung Stevia, wie beispielsweise *Stevia rebaudiana,* gewonnenen Extrakte und die in solchen Extrakten enthaltenen Glucoside, insbesondere die Diterpenglucoside vom Kauren-Typ, die auch als isolierte Süßstoffe vorliegen können, wie beispielsweise Steviosid, Rebaudioside, Dulcoside etc., sowie Mischungen solcher aus Stevia-Pflanzen gewonnener Süßstoffe.

Stevia-Extrakte können aus Pflanzenmaterial von Stevia-Pflanzen durch Extraktion, beispielsweise mit Wasser oder Ethanol, gewonnen werden. Im Rahmen der vorliegenden Erfindung können Rohextrakte aus Stevia-Pflanzen(material) oder Fraktionen der Extrakte, in denen die jeweils gewünschte(n) Substanz(en) angereichert enthalten ist (sind), oder die aus den Extrakten isolierten Süßstoffe verwendet werden. Die erfindungsgemäß zum Einsatz kommenden Stevia-Extrakte können als Flüssigkeit oder in fester Form, beispielsweise als Pulver, vorliegen.

Thaumatin ist ein Protein mit hoher Süßkraft. Es kann aus Früchten bzw. Samenkapseln der Katemfe-Pflanze isoliert werden. Da das Thaumatin-Gen leicht geklont und beispielsweise in Mikroorganismen eingeschleust werden kann, kann der Süßstoff auch gentechnisch produziert werden. Im Rahmen der vorliegenden Erfindung kann sowohl natürlich vorkommendes Thaumatin als auch der gentechnisch hergestellte Süßstoff zum Einsatz kommen.

In einer weiteren Ausgestaltung der Erfindung wird ein Futtermittel vorgeschlagen, das zur Appetit- und/oder Leistungsförderung bei Nutztieren eine Süßstoffkombination enthält, die zumindest Naringin-DC und ein Saccharin aufweist. Erfindungsgemäß kann die in dem Futtermittel enthaltene Süßstoffkombination einen weiteren Süßstoff enthalten, der vorzugsweise aus Stevia-Extrakten und Thaumatin ausgewählt ist.

Das Futtermittel kann alle herkömmlichen Futtermittelbestandteile enthalten, die in der Tierhaltung üblich sind. Dazu gehören beispielsweise stärkehaltige Bestandteile, wie Getreide und Getreideprodukte; Proteine; Aminosäuren, insbesondere essentielle Aminosäuren; Vitamine; Enzyme; mineralische Zuschlagstoffe, wie Spurenelemente, Phosphate, Salze und Kalk; und Aromastoffe. Die Proteine können natürlichen Ursprungs sein oder als Präparate zugesetzt werden. Die Aminosäuren können industriell hergestellte freie Aminosäuren oder ihre chemisch definierten Analoga (Salze) oder Konzentrate sein, die durch Reinigung und Anreicherung aus natürlichen Quellen oder durch chemische Synthese oder Fermentation hergestellt wurden. Bei Bedarf können dem Futtermittel auch weitere Zusatzstoffe, wie beispielsweise medikamentös wirkende Stoffe, zugesetzt werden.

In einer weiteren Ausgestaltung der Erfindung wird ein Futtermittelzusatz zur Herstellung eines Futtermittels vorgeschlagen, welcher Futtermittelzusatz zur Appetit- und/oder Leistungsförderung bei Nutztieren eine Süßstoffkombination enthält, die zumindest Naringin-DC und ein Saccharin umfasst. Erfindungsgemäß kann die in dem Futtermittelzusatz enthaltene Süßstoffkombination einen weiteren Süßstoff enthalten, der vorzugsweise aus Stevia-Extrakten und Thaumatin ausgewählt ist.

Unter einem Futtermittelzusatz werden in der vorliegenden Beschreibung beispielsweise Vormischungen (Premixe), Mineralfutter oder Ergänzungsfuttermittel zur Zubereitung von Futtermitteln verstanden, aber auch eine erfindungsgemäße appetit- und/oder leistungsfördernde Süßstoffkombination allein. Je nach der Art des Futtermittelzusatzes können außer einer erfindungsgemäßen Süßstoffkombination ein oder mehrere Futtermittelbestandteile enthalten sein, die aus Getreide, Getreideprodukten, Proteinen, Aminosäuren, Vitaminen, Enzymen, mineralischen Zuschlagstoffen, Aromastoffen etc. ausgewählt sind. Es können auch medikamentös wirkende Stoffe in dem Futtermittelzusatz enthalten sein.

Das Saccharin, das in dem Futtermittel oder Futtermittelzusatz gemäß der Erfindung enthalten ist, kann aus Saccharin und seinen Alkalimetall- oder Erdalkalimetallsalzen, wie beispielsweise Na-, K- oder Ca-Saccharin, ausgewählt sein, wobei unter Saccharin und/oder Saccharin-Salzen sowohl wasserfreie Formen als auch Hydrate verstanden werden.

Die Stevia-Extrakte, die in dem Futtermittel oder Futtermittelzusatz gemäß der Erfindung enthalten sein können, sind wie oben definiert.

Gemäß einem Merkmal der Erfindung kann ein Futtermittel oder Futtermittelzusatz ein Saccharin in einer Menge bis zu 500 mg und Naringin-DC in einer Menge von 0,1 bis 1000 mg, vorzugsweise 1 bis 100 mg, pro Kilogramm Futtermittel enthalten.

Gemäß einem anderen Merkmal der Erfindung kann ein Futtermittel oder Futtermittelzusatz ein Saccharin in einer Menge bis zu 500 mg, Naringin-DC in einer Menge von 0,1 bis 1000 mg, vorzugsweise 1 bis 100 mg, und Stevia-Extrakt in einer Menge von 0,1 bis 100 mg, vorzugsweise 1 bis 100 mg, pro Kilogramm Futtermittel enthalten.

Die Erfindung betrifft auch die Verwendung einer appetit- und/oder leistungsfördemden Süßstoffkombination zur Herstellung eines Futtermittels, wie es oben beschrieben ist, oder zur Herstellung eines Futtermittelzusatzes, wie er oben beschrieben ist, wobei die Süßstoffkombination zumindest Naringin-DC und ein Saccharin, wie oben definiert, enthält. Erfindungsgemäß kann eine Süßstoffkombination verwendet werden, die einen weiteren Süßstoff enthält, der vorzugsweise aus Stevia-Extrakten, wie oben definiert, und Thaumatin ausgewählt ist.

Bei vergleichenden Fütterungsversuchen unter Verwendung der herkömmlichen Kombination von Saccharin und Neohesperidin-Dihydrochalcon einerseits und Verwendung einer erfindungsgemäßen Süßstoffkombination - beispielsweise Naringin-DC und Na-Saccharin - andererseits wurde festgestellt, dass die Versuchstiere dem die erfindungsgemäße Süßstoffkombination enthaltenden Futtermittel gegenüber dem die herkömmliche Süßstoffkombination enthaltenden Futtermittel eindeutig den Vorzug gaben. Das Verhältnis liegt bei etwa 2/3 zu 1/3 zugunsten der erfindungsgemäßen Süßstoffkombination. Dies stellt einen für den Fachmann überraschenden, vorteilhaften Effekt der Erfindung dar.

Die gesamte Futteraufnahme war bei Verwendung einer erfindungsgemäßen Süßstoffkombination geringer als bei Verwendung der oben genannten herkömmlichen Süßstoffkombination. Dennoch hatten die Versuchstiere am Ende der Fütterungsperiode ein höheres Gewicht, was eine deutlich bessere Futterausnutzung darstellt; d.h. bei einer Verminderung der Futteraufnahme wird pro Tier mehr Gewicht erzielt. Die Futterausnutzung bei Verwendung eines eine erfindungsgemäße Süßstoffkombination enthaltenden Futtermittels ist gegenüber der Kombination von Neohesperidin-Dihydrochalcon mit Saccharin um etwa 15 % verbessert. Dies stellt für den Tierzüchter einen bedeutenden Wirtschaftlichkeitsfaktor und somit einen weiteren Vorteil der Erfindung dar.

Es ist anzunehmen, dass der Einsatz von Naringin-DC allein als Tierfutteradditiv - wie der Einsatz anderer Süßstoffe auch - eine zumindest appetitfördernde Wirkung hat. Die erfindungsgemäßen Vorteile werden damit jedoch nicht erzielt und die Kosten sind beträchtlich höher als bei Verwendung einer Süßstoffkombination.

Die Erfindung wird nachstehend anhand von Ausführungsbeispielen detaillierter beschrieben.

### BEISPIELE

Die nachstehend angeführten Beispiele zeigen, dass durch den Einsatz einer Süßstoffkombination aus einem Saccharin und Naringin-DC, die gegebenenfalls zusammen mit einem weiteren Süßstoff, wie beispielsweise einem Stevia-Extrakt, verwendet wird, eine Erhöhung der tierischen Leistungen und/oder der Futterakzeptanz im Vergleich zur Leistung und/oder Futterakzeptanz bei Verwendung einer herkömmlichen Futtermischung mit Na-Saccharin/Neohesperidin-Dihydrochalcon (NHDC) oder bei Verwendung eines herkömmlichen Futtermittels ohne Süßstoff zu erzielen ist.

### Beispiel 1:

Den Ferkeln von 14 Würfen (149 Ferkel) wurde ab dem 7. Lebenstag ein Prestarter angeboten, welcher entweder keinen Süßstoff (Kontrolle), einen Süßstoff auf Basis Saccharin/NHDC (Vergleich) oder ein erfindungsgemäßes Produkt auf Basis Saccharin/Naringin-DC oder auf Basis Saccharin/Naringin-DC/Stevia-Extrakt enthielt. Eine Woche nach dem Absetzen wurden die Tiere auf ein Ferkelaufzuchtfutter mit jeweils einer der drei Fütterungsvarianten umgestellt. Ab dem 22. Tag nach Absetzen erhielten alle Tiere ein unsupplementiertes Futter.

In der folgenden Tabelle 1 sind die Ergebnisse aufgeführt.

**Tabelle 1**

| | Phase 1: 7. Tag bis 7 Tage nach Absetzen | | Phase 2: 7. Tag bis 22. Tag nach Absetzen | |
|---|---|---|---|---|
| Süßstoffkombination | Futteraufnahme/Tier in Phase 1, kg | TZ* g/Tag | Futteraufnahme/Tier in Phase 2, kg | TZ* g/Tag |
| keine (Kontrolle) | 1,62 | 207 | 5,06 | 254 |
| 150 mg Saccharin + 0,75 mg NHDC pro kg Futter (Vergleich) | 1,55 | 203 | 5,10 | 256 |
| 150 mg Saccharin + 0,45 mg Naringin-DC + 4,5 mg Stevia-Extrakt pro kg Futter (Erfindung) | 1,48 | 236 | 5,02 | 298 |
| 150 mg Saccharin + 1,35 mg Naringin-DC pro kg Futter (Erfindung) | 1,51 | 225 | 5,09 | 280 |

| | | | | |
|---|---|---|---|---|
| * TZ = Tageszunahme | | | | |

Es zeigte sich, dass die Tiere mit Saccharin/Naringin-DC- bzw. mit Saccharin/Naringin-DC/Stevia-Extrakt-Ergänzung früher Futter aufnehmen und auch während der kritischen Absetzphase bessere Leistungen zeigen. Die Tiere behielten auch nach Absetzen des Futters bis zum Ende der Aufzucht ihren Vorsprung gegenüber den anderen Gruppen. Die mittleren Endgewichte betrugen:

| | |
|---|---|
| 26,73 kg | Kontrolle |
| 26 kg | Saccharin/NHDC (Vergleich). |
| 27,52 kg | Naringin-DC/Saccharin, |
| 27,69 kg | Saccharin/Naringin-DC/Stevia-Extrakt. |

Die Futterverwertung wurde durch die erfindungsgemäße Süßstoffkombination ebenfalls deutlich verbessert, beispielsweise im Falle von Saccharin/Naringin-DC/Stevia-Extrakt von 0,73 auf 0,61 Futtereinheiten bis zum 22. Tag nach Ende der Säugezeit:
Die Leistungen vom Tag 7 nach Geburt bis Tag 22 nach Ende der Säugeperiode sind in der folgenden Tabelle 2 gezeigt.

**Tabelle 2**

| Süßstoffkombination | Zuwachs | Futter | Futteraufwand inkl. Säugezeit |
|---|---|---|---|
| keine (Kontrolle) | 9,14 kg | 6,68 kg | 0,73 |
| Saccharin/NHDC (Vergleich) | 9,22 kg | 6,65 kg | 0,72 |
| Saccharin/Naringin-DC/Stevia-Extrakt (Erfindung) | 10,73 kg | 6,50 kg | 0,61 |
| Saccharin/Naringin-DC (Erfindung) | 10,5 kg | 6,6 kg | 0,63 |

Erstaunlich ist, dass die mit einem erfindungsgemäßen Futter gefütterten Tiere nicht der Mehrleistung entsprechend mehr Futter aufnahmen. Entscheidend ist hier wohl die frühe Futteraufnahme gerade auch nach dem Absetzen und womöglich eine bessere Nährstoffausnutzung, wie sie in der Literatur für einige Wirkstoffe in Bezug auf Extrakte aus Grapefruitprodukten beschrieben ist.

### Beispiel 2:

80 Absetzferkel wurden gleichmäßig auf 4 Buchten verteilt, und es wurden ihnen jeweils zwei Futter angeboten. Das Futter 1 enthielt als Süßstoff eine handelsübliche Mischung aus Na-Saccharin (150 mg/kg Futter) und NHDC (0,75 mg/kg Futter), während das Futter 2 eine Mischung aus Na-Saccharin (150 mg/kg Futter), Naringin-DC (0,45 mg/kg Futter) und Stevia-Extrakt (4,5 mg/kg Futter) enthielt. In jeder Bucht standen zwei Futterautomaten zur freien Auswahl zur Verfügung. In der folgenden Tabelle 3 sind die Ergebnisse aufgeführt.

**Tabelle 3**

| | Startgewicht (kg) / Tier | Futtermenge/Wo che/Bucht (kg) Saccharin/NHDC | Futtermenge/Wo che/Bucht (kg) Saccharin/Naringin-DC/Stevia-Extrakte | Futtermenge/Wo che/Bucht (kg) Saccharin/Naringin-DC |
|---|---|---|---|---|
| Woche 1 | 9,32 | 35,6 | 50,3 | 50 |
| Woche 2 | 9,67 | 117,1 | 135,4 | 136 |
| Woche 3 | 12 | 175,2 | 293,9 | 290 |
| Woche 4 | 15 | 260,9 | 347,8 | 340 |
| Woche 5 | 18,9 | 321,6 | 430,9 | 432 |
| Woche 6 | 23,45 | 235,9 | 276,4 | 270 |
| Gesamt | 26,26 | 1146,3 | 1534,7 | 1518 |

Es zeigt sich, dass das eine erfindungsgemäße Süßstoffkombination enthaltende Futter über den gesamten Versuchszeitraum besser gefressen wird als ein herkömmliches, eine herkömmliche Mischung aus Saccharin und NHDC enthaltendes Futter. Die Mischung der Süßkraft von Saccharin und Naringin-DC sowie gegebenenfalls einem weiteren Süßstoff stellt somit einen wesentlichen Fortschritt in der Ferkelfütterung dar.

## Patentansprüche

1. Futtermittel, das einen oder mehrere herkömmliche Futtermittelbestandteile enthält, die ausgewählt sind aus Getreide, Getreideprodukten, Proteinen, Aminosäuren, Vitaminen, Enzymen, mineralischen Zuschlagstoffen und Aromastoffen, oder Futtermittelzusatz zur Herstellung solcher Futtermittel, **dadurch gekennzeichnet, dass** eine Süßstoffkombination zur Appetit- und/oder Leistungsförderung enthalten ist, die zumindest Naringin-Dihydrochalcon (Naringin-DC) und ein Saccharin umfasst.

2. Futtermittel oder Futtermittelzusatz nach Anspruch 1, **dadurch gekennzeichnet, dass** das Saccharin in einer Menge bis zu 500 mg und Naringin-DC in einer Menge von 0,1 bis 1000 mg, vorzugsweise 1 bis 100 mg, pro Kilogramm Futtermittel enthalten ist.

3. Futtermittel oder Futtermittelzusatz nach Anspruch 1, **dadurch gekennzeichnet, dass** die Süßstoffkombination einen weiteren Süßstoff enthält, der vorzugsweise aus Stevia-Extrakten und Thaumantin ausgewählt ist.

4. Futtermittel oder Futtermittelzusatz nach Anspruch 3, **dadurch gekennzeichnet, dass** ein Saccharin, Naringin-DC und Stevia-Extrakt enthalten sind.

5. Futtermittel oder Futtermittelzusatz nach Anspruch 4, **dadurch gekennzeichnet, dass** das Saccharin in einer Menge bis zu 500 mg, Naringin-DC in einer Menge von 0,1 bis 1000 mg, vorzugsweise 1 bis 100 mg, und Stevia-Extrakt in einer Menge von 0,1 bis 100 mg, vorzugsweise 1 bis 100 mg, pro Kilogramm Futtermittel enthalten ist.

6. Futtermittel oder Futtermittelzusatz nach einem der Ansprüche 1 bis 5, **dadurch gekennzeichnet, dass** das Saccharin aus Saccharin und seinen Alkalimetall- oder Erdalkalimetallsalzen ausgewählt ist.

7. Futtermittel oder Futtermittelzusatz nach Anspruch 6, **dadurch gekennzeichnet, dass** das Saccharin aus Na-Saccharin, K-Saccharin und Ca-Saccharin ausgewählt ist.

8. Futtermittelzusatz nach einem der Ansprüche 1 bis 7, **dadurch gekennzeichnet, dass** weiters ein oder mehrere Futtermittelbestandteile enthalten sind, die ausgewählt sind aus Getreide, Getreideprodukten, Proteinen, Aminosäuren, Vitaminen, Enzymen, mineralischen Zuschlagstoffen und Aromastoffen.

9. Verwendung einer Süßstoffkombination zur Appetit- und Leistungsförderung bei Nutztieren, wobei die Süßstoffkombination zumindest Naringin-DC und ein Saccharin umfasst.

10. Verwendung nach Anspruch 9, wobei das Saccharin in einer Menge bis zu 500 mg und Naringin-DC in einer Menge von 0,1 bis 1000 mg, vorzugsweise 1 bis 100 mg, pro Kilogramm Futtermittel enthalten ist.

11. Verwendung nach Anspruch 9, wobei die Süßstoffkombination einen weiteren Süßstoff enthält, der vorzugsweise aus Stevia-Extrakten und Thaumantin ausgewählt ist.

12. Verwendung nach Anspruch 11, wobei die Süßstoffkombination ein Saccharin, Naringin-DC und Stevia-Extrakt enthält.

13. Verwendung nach Anspruch 12, **dadurch gekennzeichnet, dass** das Saccharin in einer Menge bis zu 500 mg, Naringin-DC in einer Menge von 0,1 bis 1000 mg, vorzugsweise 1 bis 100 mg, und Stevia-Extrakt in einer Menge von 0,1 bis 100 mg, vorzugsweise 1 bis 100 mg, pro Kilogramm Futtermittel enthalten ist.

14. Verwendung nach einem der Ansprüche 9 bis 13, wobei das Saccharin aus Saccharin und seinen Alkalimetall- oder Erdalkalimetallsalzen ausgewählt ist.

15. Verwendung nach Anspruch 14, wobei das Saccharin aus Na-Saccharin, K-Saccharin und Ca-Saccharin ausgewählt ist.

16. Verwendung einer Süßstoffkombination, die zumindest Naringin-DC und ein Saccharin enthält, zur Herstellung eines Futtermittels gemäß einem der Ansprüche 1 bis 7 oder zur Herstellung eines Futtermittelzusatzes gemäß einem der Ansprüche 1 bis 8.

17. Verwendung einer Süßstoffkombination, die Naringin-DC, ein Saccharin und einen aus Stevia-Extrakten und Thaumantin ausgewählten weiteren Süßstoff enthält, zur Herstellung eines Futtermittels gemäß einem der Ansprüche 3 bis 7 oder zur Herstellung eines Futtermittelzusatzes gemäß einem der Ansprüche 3 bis 8.

## Claims

1. Feedstuff which contains one or more customary feedstuff components which are selected from cereals, cereal products, proteins, amino acids, vitamins, enzymes, mineral additives and flavourings, or feedstuff additives for producing such feedstuffs, **characterized in that** a sweetener combination is present for appetite and/or performance enhancement, which sweetener combination comprises at least naringin dihydrochalcone (naringin-DC) and a saccharin.

2. Feedstuff or feedstuff additive according to Claim 1, **characterized in that** the saccharin is present in an amount up to 500 mg and naringin-DC in an amount from 0.1 to 1000 mg, preferably 1 to 100 mg, per kilogram of feedstuff.

3. Feedstuff or feedstuff additive according to Claim 1, **characterized in that** the sweetener combination contains a further sweetener that is preferably selected from stevia extracts and thaumatin.

4. Feedstuff or feedstuff additive according to Claim 3, **characterized in that** a saccharin, naringin-DC and stevia extract are present.

5. Feedstuff or feedstuff additive according to Claim 4, **characterized in that** the saccharin is present in an amount up to 500 mg, naringin-DC in an amount from 0.1 to 1000 mg, preferably 1 to 100 mg, and stevia extract in an amount from 0.1 to 100 mg, preferably 1 to 100 mg, per kilogram of feedstuff.

6. Feedstuff or feedstuff additive according to any one of Claims 1 to 5, **characterized in that** the saccharin is selected from saccharin and alkali metal salts or alkaline earth metal salts thereof.

7. Feedstuff or feedstuff additive according to Claim 6, **characterized in that** the saccharin is selected from Na-saccharin, K-saccharin and Ca-saccharin.

8. Feedstuff additive according to any one of Claims 1 to 7, **characterized in that**, in addition, one or more feedstuff components are present that are selected from cereals, cereal products, proteins, amino acids, vitamins, enzymes, mineral additives and flavourings.

9. Use of a sweetener combination for appetite and performance enhancement in farm animals, wherein the sweetener combination comprises at least naringin-DC and a saccharin.

10. Use according to Claim 9, wherein the saccharin is present in an amount up to 500 mg and naringin-DC is present in an amount from 0.1 to 1000 mg, preferably 1 to 100 mg, per kilogram of feedstuff.

11. Use according to Claim 9, wherein the sweetener combination contains a further sweetener which is preferably selected from stevia extracts and thaumatin.

12. Use according to Claim 11, wherein the sweetener combination contains a saccharin, naringin-DC and stevia extract.

13. Use according to Claim 12, **characterized in that** the saccharin is present in an amount up to 500 mg, naringin-DC in an amount from 0.1 to 1000 mg, preferably 1 to 100 mg, and stevia extract in an amount from 0.1 to 100 mg, preferably 1 to 100 mg, per kilogram of feedstuff.

14. Use according to any one of Claims 9 to 13, wherein the saccharin is selected from saccharin and alkali metal salts or alkaline earth metal salts thereof.

15. Use according to Claim 14, wherein the saccharin is selected from Na-saccharin, K-saccharin and Ca-saccharin.

16. Use of a sweetener combination that contains at least naringin-DC and a saccharin for producing a feedstuff according to any one of Claims 1 to 7 or for producing a feedstuff additive according to any one of Claims 1 to 8.

17. Use of a sweetener combination that contains naringin-DC, a saccharin and a further sweetener selected from stevia extracts and thaumatin for producing a feedstuff according to any one of Claims 3 to 7 or for producing a feedstuff additive according to any one of Claims 3 to 8.

## Revendications

1. Aliment pour animaux, qui contient un ou plusieurs constituant(s) ordinaire(s) des aliments pour animaux, choisi(s) parmi les céréales, produits à base de céréales, protéines, acides aminés, vitamines, enzymes, sels minéraux ajoutés et substances aromatiques, ou adjuvant d'aliments pour animaux, destiné à la préparation de tels aliments pour animaux, **caractérisé en ce qu'**il contient une combinaison d'édulcorants, conçue pour améliorer l'appétit et/ou le rendement, qui comprend au moins de la dihydrochalcone de naringine (naringine-DC) et une saccharine.

2. Aliment pour animaux, ou adjuvant d'aliments pour animaux, conforme à la revendication 1, **caractérisé en ce qu'**il contient de la saccharine en une quantité pouvant aller jusqu'à 500 mg, et de la dihydrochalcone de naringine en une quantité de 0,1 à 1000 mg et de préférence de 1 à 100 mg, par kilogramme d'aliment pour animaux.

3. Aliment pour animaux, ou adjuvant d'aliments pour animaux, conforme à la revendication 1, **caractérisé en ce que** la combinaison d'édulcorants comprend un autre édulcorant, qui est de préférence choisi parmi des extraits de stevia et de la thaumatine.

4. Aliment pour animaux, ou adjuvant d'aliments pour animaux, conforme à la revendication 3, **caractérisé en ce qu'**il contient une saccharine, de la dihydrochalcone de naringine, et un extrait de stevia.

5. Aliment pour animaux, ou adjuvant d'aliments pour animaux, conforme à la revendication 4, **caractérisé en ce qu'**il contient de la saccharine en une quantité pouvant aller jusqu'à 500 mg, de la dihydrochalcone de naringine, en une quantité de 0,1 à 1000 mg et de préférence de 1 à 100 mg, et de l'extrait de stevia en une quantité de 0,1 à 100 mg, et de préférence de 1 à 100 mg, par kilogramme d'aliment pour animaux.

6. Aliment pour animaux, ou adjuvant d'aliments pour animaux, conforme à l'une des revendications 1 à 5, **caractérisé en ce que** la saccharine est choisie parmi la saccharine proprement dite et ses sels de métal alcalin ou alcalino-terreux.

7. Aliment pour animaux, ou adjuvant d'aliments pour animaux, conforme à la revendication 6, **caractérisé en ce que** la saccharine est choisie parmi les sels de sodium, de potassium et de calcium de la saccharine proprement dite.

8. Adjuvant d'aliments pour animaux, conforme à l'une des revendications 1 à 7, **caractérisé en ce qu'**il contient en outre un ou plusieurs constituant(s) d'aliments pour animaux, choisi(s) parmi les céréales, produits à base de céréales, protéines, acides aminés, vitamines, enzymes, sels minéraux ajoutés et substances aromatiques.

9. Utilisation, pour améliorer l'appétit et/ou le rendement chez des animaux d'élevage, d'une combinaison d'édulcorants qui comprend au moins de la dihydrochalcone de naringine (naringine-DC) et une saccharine.

10. Utilisation conforme à la revendication 9, pour laquelle la saccharine est contenue en une quantité pouvant aller jusqu'à 500 mg, et la dihydrochalcone de naringine est contenue en une quantité de 0,1 à 1000 mg et de préférence de 1 à 100 mg, par kilogramme d'aliment pour animaux.

11. Utilisation conforme à la revendication 9, pour laquelle la combinaison d'édulcorants comprend un autre édulcorant, qui est de préférence choisi parmi des extraits de stevia et de la thaumatine.

12. Utilisation conforme à la revendication 11, pour laquelle la combinaison d'édulcorants comprend une saccharine, de la dihydrochalcone de naringine, et un extrait de stevia.

13. Utilisation conforme à la revendication 12, **caractérisée en ce que** la saccharine est contenue en une quantité pouvant aller jusqu'à 500 mg, la dihydrochalcone de naringine est contenue en une quantité de 0,1 à 1000 mg et de préférence de 1 à 100 mg, et l'extrait de stevia est contenu en une quantité de 0,1 à 100 mg et de préférence de 1 à 100 mg, par kilogramme d'aliment pour animaux.

14. Utilisation conforme à l'une des revendications 9 à 13, pour laquelle la saccharine est choisie parmi la saccharine proprement dite et ses sels de métal alcalin ou alcalino-terreux.

15. Utilisation conforme à la revendication 14, pour laquelle la saccharine est choisie parmi les sels de sodium, de potassium et de calcium de la saccharine proprement dite.

16. Utilisation d'une combinaison d'édulcorants qui comprend au moins de la dihydrochalcone de naringine et une saccharine, en vue de la préparation d'un aliment pour animaux, conforme à l'une des revendications 1 à 7, ou en vue de la préparation d'un adjuvant d'aliments pour animaux, conforme à l'une des revendications 1 à 8.

17. Utilisation d'une combinaison d'édulcorants qui comprend au moins de la dihydrochalcone de naringine, une saccharine et un autre édulcorant choisi parmi des extraits de stevia et de la thaumatine, en vue de la préparation d'un aliment pour animaux, conforme à l'une des revendications 3 à 7, ou en vue de la préparation d'un adjuvant d'aliments pour animaux, conforme à l'une des revendications 3 à 8.
